Europäisches Patentamt

European Patent Office

Office européen des brevets

(11) Veröffentlichungsnummer: **0 170 982**
A1

(12) **EUROPÄISCHE PATENTANMELDUNG**

(21) Anmeldenummer: **85109273.4**

(22) Anmeldetag: **24.07.85**

(51) Int. Cl.⁴: **A 61 F 2/36**

(30) Priorität: **26.07.84 DE 3427500**

(43) Veröffentlichungstag der Anmeldung: **12.02.86**
**Patentblatt 86/7**

(84) Benannte Vertragsstaaten: **AT BE CH DE FR GB IT LI LU NL SE**

(71) Anmelder: **Jerkovic, Ivica, Stockacher Strasse 63, D-7200 Tuttlingen (DE)**

(72) Erfinder: **Jerkovic, Ivica, Stockacher Strasse 63, D-7200 Tuttlingen (DE)**

(74) Vertreter: **Hiebsch, Gerhard F., Dipl.-Ing., Erzbergerstrasse 5A Postfach 464, D-7700 Singen 1 (DE)**

(54) **Gelenkendoprothese, insbesondere Hüftgelenkprothese.**

(57) Eine Gelenkendoprothese, insbesondere eine Hüftgelenkprothese, deren Schaft sich vom distalen freien Ende aus konisch erweitert und aus einem Schafthauptteil und einem lateralen, keilförmigen Gegenstück besteht, welches am Schafthauptteil längsverschiebbar ist, soll es bei zementfreier oder zumindest zementarmer Verankerung im Knochen unter anderem ermöglichen, daß harte Stöße von der Schaftverankerung ferngehalten werden und einer Schaftlockerung selbsttätig entgegengewirkt wird.

Hierzu steht das Gegenstück (3) unter einem in Richtung zum freien Ende wirksamen Druck von am Schafthauptteil (2) abgestützten Federelementen (42, 43) und ist gegenüber dem Schafthauptteil (2) in seiner Ausgangsstellung lösbar verriegelt. Zudem soll das keilförmige Gegenstück (3) am Schafthauptteil (2) geführt und von einer Abdeckschiene (4) lateral und beidseitig umschlossen sein.

# DIPL.-ING. GERHARD F. HIEBSCH

PATENTANWALT

EUROPEAN PATENT ATTORNEY

Ivica Jerković
===============

Stockacher Straße 63
====================

7200 Tuttlingen
===============

D-7700 Singen 1
Erzbergerstraße 5a
Telegr./Cables:
Bodenseepatent
Telex 793850
Telefon (07731) 63075
                63076

Mein Zeichen   J-147/EPA
My file

I/ke

Datum / Date

Gelenkendoprothese, insbesondere Hüftgelenkprothese

Die Erfindung betrifft eine Gelenkendoprothese, die sich in einer bevorzugten Ausgestaltung insbesondere als Hüftgelenkprothese eignet. Eine derartige Gelenkprothese ist durch die europäische Patentanmeldung 0085 147 bekannt geworden, bei welcher der Schaft sich vom distalen freien Ende aus konisch erweitert und aus einem Schafthauptteil und einem lateralen, keilförmigen Gegenstück besteht, welches am Schafthauptteil längsverschiebbar ist.

Bei der bekannten Gelenkendoprothese soll der Schaft zementfrei oder zumindest zementarm im Knochen verankert werden, wobei die tragende Abstützung durch Verklemmen des Schaftes im Knochen erfolgt. Das Fixieren und Verankern erfolgt dabei in der Weise, daß der Schafthauptteil zuerst in den vorbereiteten Knochen eingeschlagen und darauf das keilförmige Gegenstück eingesetzt und am Schafthauptteil längsverschiebbare gleitend ebenfalls eingeschlagen wird, so daß ein Verkeilen des ganzen Schaftes im Knochen erreicht wird.

Ausgehend von einer derartigen Gelenkendoprothese liegt der Erfindung die Aufgabe zugrunde, bei einer ebenfalls zementfreien oder zumindest zementarmen Verankerung im Knochen zu erreichen, daß harte Stöße von der Schaftverankerung ferngehalten werden und daß einer Schaftlockerung selbsttätig entgegengewirkt wird, und weiterhin, daß eine sich anbahnende Überbeanspruchung des Knochens und der Gelenkprothese selbst dem Prothesenträger bewußt gemacht werden kann.

Zur Lösung dieser Aufgabenstellungen wird erfindungsgemäß bei einer Gelenkendoprothese der eingangs genannten Gattung vorgeschlagen, daß das Gegenstück unter einem in Richtung zum freien Ende wirksamen Druck von am Schafthauptteil abgestützten Federelementen steht und gegenüber dem Schafthauptteil in seiner Ausgangsstellung lösbar verriegelt ist.

Dabei ist die weitere Anordnung so getroffen, daß das keilförmige Gegenstück am Schafthauptteil geführt und von einer Abdeckschiene lateral und beidseitig umschlossen ist. Insbesondere weist die Abdeckschiene einen U-förmigen Querschnitt auf, so daß es das Gegenstück umschließt und den Schafthauptteil beidseitig teilweise umgreift.

Ein weiteres wesentliches Merkmal der Erfindung besteht darin, daß der Schafthauptteil mit einem Schaft-Federwiderlager und einer Schaft-Federnut zur Aufnahme von Druckfedern versehen ist, und daß auch das Gegenstück eine Federaufnahme und ein Federlager aufweist. Weiterhin kann das keilförmige Gegenstück in einer Führungsnut des Schafthaupteils an diesem geführt sein.

Die Wirkungsweise dieses Prothesenschafts besteht darin, daß in entriegelter Stellung das keilförmige Gegenstück unter der Federwirkung entlang dem Schafthauptteil ins Gleiten gebracht wird. Dabei tritt die Nase des Gegenstückvorstoßers aus dem Ende des Schafthauptteils hervor und wird durch das Knochenmark geführt. Durch diese Bewegung, die parallel zur Mittellinie des eigentlichen Prothesenschaftes verläuft, kommt es zur Kompression auf die umliegende harte Knochensubstanz. Vorteilhaft wirkt sich die Anordnung der Abdeckschiene aus, weil diese in ihrer Lage zum Schafthauptteil verbleibt und daher an der Gleitbewegung des Gegenstücks nicht teilnimmt, durch welche dieses und der Schafthauptteil auseinandergedrückt werden. Dadurch entsteht eine Umfangs-und Längenvergrösserung des gesamten Prothesenschaftes.

Ein weiteres bedeutsames Merkmal der Erfindung ist darin zu sehen, daß mittels einer Einzahndoppelgelenksperre, bestehend aus einem Sperrhebel, der durch eine Gelenksperrlasche und zwei Gelenkbolzen mit dem keilförmigen Gegenstück auslenkbar verbunden ist, wahlweise drei Ver-oder Entriegelungsstellungen des Sperrhebels gegenüber dem Schafthauptteil einstellbar sind, in denen beide Teile gegeneinander verriegelt sind, in einer zweiten Stellung das Einsetzen einer Spongiosaschraube möglich ist, und in einer dritten Stellung des Gegenstück entriegelt ist, so daß es unter der Federwirkung längsverschiebbar ist.

Wesentliche Gestaltungsmerkmale beziehen sich auf den Prothesenkopf. Der Schafthauptteil endet in einem Prothesenkragen, der den Prothesenkopfsockel umringt und der eine ringförmige Senkung aufweist, aus deren Mitte der zylindrische Einsteckbolzen herausragt, der mit einer Ringfedernut zur Aufnahme einer Ringfeder versehen ist, die zur Sicherung gegen Herausziehen des auf den Einsteckbolzen aufgesteckten Prothesenkopfes dient. Der Prothesenkragen kann außerdem mit Führungsmitteln für das keilförmige Gegenstück und die Abdeckschiene versehen sein, und zwar mit beidseitigen Führungsnocken und an deren Innenseiten eingeschnittenen Führungsnuten. Außerdem weist sie zwei axial zueinander angeordnete, die beiden Stege des U-förmigen Profils durchsetzende Langlochbohrungen auf. Mittels zweier Schrauben, die in die Langlochbohrungen eingreifen und beidseitig in die Führungsnocken eingeschraubt sind, kann die Abdeckschiene gelenkig und um einen kleinen Betrag auslenkbar mit dem Prothesenkragen verbunden sein.

Durch diese gelenkige Verbindung ist es möglich, die Abdeckschiene rechtwinklig vom Schafthauptteil abzuspreizen, da sie sich in Richtung der genannten Langlochbohrungen auseinanderschieben läßt. Der Schafthauptteil und die Abdeckschiene können daher wie ein Zirkel aufgeklappt werden, wodurch ein leichtes Einschieben des keilförmigen Gegenstücks ermöglicht wird.

Das keilförmige Gegenstück endet in einem Gegenstückvorstoßer, der mit einem Federlager in Form einer Sackbohrung zur Aufnahme einer Schrauben-Druckfeder versehen ist. Diese findet ihr Widerlager im Schaft, welches als eine zu einem Drittel ihres Durchmessers auf ihrer ganzen Länge freigelegte Sackbohrung gestaltet ist. Die Druckfeder stützt sich daher einerseits in der Sackbohrung des Federlagers im Gegenstückvorstoßer und in der zu einem Drittel offenen Sackbohrung des Schafthauptteils ab.

Der Schafthauptteil kann ferner mit einer zweiten Schaftfedernut und das Gegenstück mit einer zweiten Federaufnahme versehen sein, in denen eine weitere Schrauben-Druckfeder gelagert ist. Diese sind so gestaltet, daß die Druckfeder mit einem Teil ihres Durchmessers in der Schaft-Federnut und mit dem anderen Teil ihres Durchmessers in der Federaufnahme des keilförmigen Gegenstücks untergebracht ist. Die beiden letztgenannten Ausnehmungen sind zueinander so angeordnet, daß sie in der Ausgangsstellung des Gegenstücks gegeneinander längsversetzt sind, jedoch eine gemeinsame Mittellinie haben. Dadurch wird erreicht, daß die Feder auf das keilförmige Gegenstück einen Druck ausübt, der höher sein soll, als der Druck der oben erstgenannten Druckfeder. Dadurch wird erreicht, daß der Gleitweg des Gegenstücks der Länge der Versetzung der beiden zuletztgenannten Ausnehmungen entspricht und die Endstellung des Gegenstücks erreicht ist, wenn sich die beiden Ausnhemungen decken.

Wie bereits erwähnt, dient der Aufsteckbolzen des Prothesenkragens zur Halterung des Prothesenkopfes. Dieser ist nach weiteren Merkmalen der Erfindung mit einem in die ringförmige Senkung des Prothesenkragens passenden Prothesenkopfsockel, mit einer hinteren Batterieaufnahmebohrung und mit einer vorderen Einsteckbolzenbohrung versehen, in welcher eine eingeschnittene bereite Federringnut angebracht ist, die eine Axialbewegung des Prothesenkopfes gegenüber der in der Ringfedernut des Einsteckbolzens eingesetzten Ringfeder ermöglicht. Weiterhin ist zwischen dem Prothesenkopfsockel und der Grundfläche der ringförmigen Senkung im Prothesenkragen ein elastischer Gummiring eingesetzt, so daß die gesamte Grundfläche des Prothesenkopfsockels auf dem Gummiring aufsitzt, umschlossen von dem Prothesenkragen. Der Gummiring dient in erster Linie dazu, Druckstöße auf den Prothesenkopf abzudämpfen.

Bei der Ausbildung derGelenkendoprothese als Hüftgelenkprothese weist der Zylindrische Einsteckbolzen mit dem Prothesensockel und Prothesenhals sowie Prothesenkopf eine axiale Neigung von in der Regel ca. 45$^{\text{o}}$ gegenüber dem Prothesenschaft auf. Dabei ist der Prothesenkopf mittels eines Gewindestiftes gegen Rotation gesichert.

Eine sehr wesentliche Teilaufgabe der Erfindung wird durch zusätzliche Merkmalskombinationen gelöst, und zwar in Verbindung mit dem weiteren Vorschlag, daß auf der Stirnfläche des Einsteckbolzens ein Gummiplättchen eingesetzt ist. Auch dieses Gummiplättchen hat wie der bereits erwähnte Gummiring unterhalb des Prothesenkopfsockels die Aufgabe, auftretende Druckstöße zu dämpfen.

Eine weitere wesentliche Bedeutung kommt diesem Gummiplättchen wie auch dem obenerwähnten Gummiring dadurch zu, daß beide Gummikörper Bestandteile eines elektrischen Schalters bilden, der dazu bestimmt ist, beim Erreichen von gerade noch zulässigen Grenzbeanspruchungen durch statischen Druck oder durch dynamische Druckstöße ein Warnsignal auszulösen, welches der Prothesenträger wahrnehmen kann.

Hierzu ist die Anordnung erfindungsgemäß so getroffen, daß zwischen der Stirnfläche des Einsteckbolzens und dem Gummiplättchen eine Unterlegscheibe aus einem elektrisch leitenden Werkstoff, und daß in eine Gewindebohrung im Zentrum des Einsteckbolzens eine Kontaktfeineinstellschraube eingesetzt ist, mit welcher die in die Batterieaufnahmebohrung des Prothesenkopfes eingesetzte Batterieeinheit bei starker Zusammenpressung des elastischen Gummirings und des Gummiplättchens in Berührung gelangt.

Mit Hilfe der Kontaktfeineinstellschraube kann die Größe der Zusammendrückung verändert werden, bei deren Erreichen diese Berührung erfolgt. Um ein ungewolltes Verdrehen zu verhindern, ist weiter vorgesehen, daß die Kontaktfeineinstellschraube durch Reibung in der engen Mittelbohrung des auf dem Einstellbolzen aufsitzenden Gummiplättchens in ihrer jeweiligen Stellung gesichert ist.

Beim Aufsitzen der Batterieeinheit auf der Kontakfeineinstellschraube wird durch diese Berührung der Stromkreis geschlossen, und mittels Kabeln wird der dadurch ausgelöste Stromstoß zu einem Signalgeber weitergeleitet. Wird nach Aufhören der Druckbelastung der Berührungskontakt wieder aufgehoben, so wird infolge des Unterbrechens des Stromkreises kein weiteres Signal ausgelöst. Als Signalgeber kann eine auf der Haut des Prothesenträgers befestigte oder aber implantierte Anschlußscheibe dienen. Wenn sich die Überlastungsignale häufen, so ist der Prothesenträger durch die Warnreize informiert, daß eine vorbestimmte Belastungsobergrenze überschritten und eine ärztliche Untersuchung notwendig wird.

Zweckmäßig ist es nach einem weiteren Vorschlag, daß der Prothesenkopf mit einer in die Batterieaufnahmebohrung und in die Einsteckbolzenbohrung eingestochene Kabelnut und einem in diesen mündenden nach außen führenden Kabelausgang versehen ist. Dadurch wird geschützte und nicht störende Kabelführung ermöglicht.

Die erfindungsgemäße Gelenkendoprothese ist in ihren Anwendungs-und Ausgestaltungsmöglichkeiten vorzugsweise als Hüftgelenkprothese konzipiert, jedoch auch für den Einsatz an anderen Gelenken geeignet. Daher ist die elektrische Reiz-Signalgebung nicht stets erforderlich, und die Prothese ist auch ohne diese Einrichtung besonders vorteilhaft, denn sie wirkt durch die zwei eing druckdämpfenden Elemente äußerst schonend udn wirkt dadurch einer Lockerung des Prothesenschafts entgegen. Der grundlegende Vorteil der erfindungsgemäßen Ausbildungsweise besteht jedoch darin, daß der Prothesenschaft einer angehenden Lockerung dadurch aktive entgegenwirkt, daß sich der Prothesenschaftumfang kontinuierlich dem Schwund der harten Knochensubstanz (z.B. Osteoporose) anpaßt. Dadurch wird ohne operative oder sonstige exteren Eingriffe immer eine festsitzende zementfreie Prothese gewährleistet.

I-147 /EPA                                               -8-

Bei einer weitere Ausbildung der erfindungsgemäßen
Gelenkendoprothese kann die Einzahndoppelgelenksperre durch eine einfachere Lösung ersetzt werden,
bei welcher die Entriegelung durch eine im Bereich
des oberen Endes des keilförmigen Gegenstücks angebrachte Halbrundnut und ein in Querrrichtung verlaufendes, mit der Halbrundnut axial übereinstimmendes Langloch in der Abdeckschiene zum Durchstecken
eines Zylinderstifts mit einer Funktionsnut zu bewirken ist, wobei der Zylinderstift in einer Bohrung
des Prothesenkragens des Schafthauptteils gelagert
ist. Dabei ermöglicht das Langloch ebenfalls ein
Aufspreizen von Schafthauptteil und Abdeckschiene,
so daß das Einschieben des keilförmigen Gegenstücks
möglich ist, wenn der Zylinderstift um 180$^{\circ}$ gedreht
wird. In Normalstellung des Zylinderstifts sind die
Teile miteinander verriegelt, so daß der Prothesenschaft eingeschlagen werden kann. In der Entriegelungsstellung wird die Halbrundnut des keilförmigen
Gegenstücks frei und dieses kann sich unter der Federwirkung gegenüber dem Schafthauptteil und der Abdeckschiene verschieben.

Im Rahmen der Erfindung kann der Gelenkendoprothesenschaft auch so ausgeführt sein, daß das Abdeckelement
den Schafthauptteil an seinem Ende umschließt, wobei
diese beiden um die Weglänge des keilförmigen Gleitelementes verlängert sind, so daß der zylindrische
Vorstoßer und seine Nase sich innerhalb des eigentlichen Prothesenschaftes bewegen und nicht durch den
Knochenmarkkanal geführt werden. Dadurch kommt es zu
einer Umfangvergrößerung des eigentlichen Gelenkendoprothesenschaftes, ohne daß er sich verlängern würde.

-9-

Eine besondere Ausführung der bereits erwähnten Einzahndoppelgelenksperre zeichnet sich dadurch aus, daß sie
weitere Einrastzähne -- also mehr als einen Einrastzahn --
anbietet, um die ständige Druckwirkung der Schraubenfeder nach selbständigem Beheben einer Schaftlockerung
zu unterbinden. Zudem besteht noch die Möglichkeit, erneute Schaftlockerungen durch externes Entsichern der
Sperre zu beheben.

Von erfindungsgemäßer Bedeutung ist auch ein dreiteiliger
Gelenkendoprothesenschaft aus Schafthauptteil, keilförmigem
Gleitelement und Abdeckelement, bei dem Federnut, Federwiderlager, Federaufnahme und Federlager fehlen. Diese
Schaftausführung vereinfacht das Einpassen bzw. erleichtert
das Implantieren des Gelenkendoprothesenschaftes in den
Femurknochen, indem man nach gleichzeitigem Einsetzen des
Schafthauptteils und des Abdeckelements das keilförmige
Gleitelement manuell dazwischentreibt. Hierdurch entsteht
ein optimales Verklemmen des eigentlichen Gelenkendoprothesenschaftes im Femurknochen.

Die erfindungsgemäße Gelenkendoprothese kann aus allen
bekannten Implantatenstählen bzw. Werkstoffen hergestellt werden, wobei Werkstoffe zu bevorzugen sind,
die nicht zum Kaltverschweißen neigen. Infolge der neuartigen Element-Kombination des Schaftaufbaues wird eine auftretende Lockerung selbsttätig behoben. Daher wird
der Prothesenträger nicht durch eine Reoperation belastet
und weiterhin werden Druckstöße in starkem Maße gedämpft.
In der bevorzugten Ausführung erlernt der Patient die zulässige Belastungsgrenze und kann sich danach verhalten.
Die Prothese kann mehrere Modelle bisheriger Prothesen
ersetzen, da sie durch Austausch des keilförmigen Gegenstücks auf verschiedene Ausgangsdurchmesser umgestellt
werden kann, und sie eignet daher auch gut für Austauschoperationen von gelockerten zementlosen oder Zementprothesen.

Die Erfindung und weitere ihrer Merkmale sind im folgenden anhand der Zeichnung weiter beschrieben und
näher erläutert. Es zeigen

Fig. 1   das obere Ende eines Femurknochens mit einer
         implantierten Gelenkendoprothese als Hüftgelenk im Längsschnitt;

Fig. 2   eine Ansicht des Prothesenkopfes in Kugelaus-
         führung;

Fig. 3   einen Querschnitt des Prothesenkopfes nach
         Fig. 2;

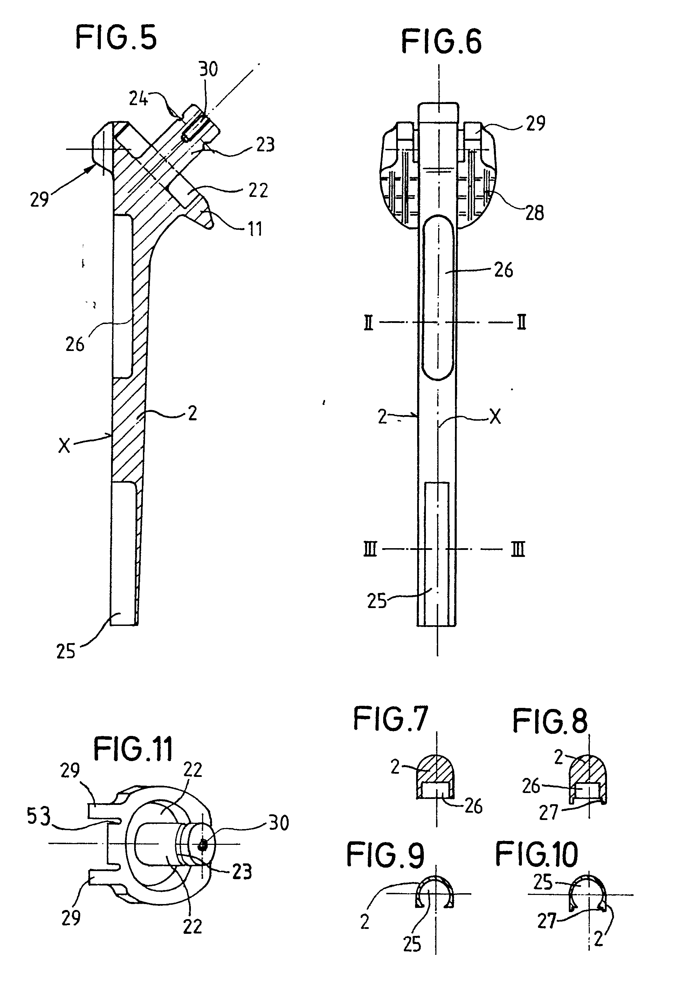

Fig. 4   einen Querschnitt durch die Batterieeinheit;

Fig. 5   einen Längsschnitt durch den Schafthauptteil;

Fig. 6   eine Ansicht des Schafthauptteils nach Fig.5;

Fig. 7   einen Querschnitt  II - II gem. Fig.6;

Fig. 8   einen Querschnitt  II - II gem. Fig.6 in einer abgewandelten Ausführung;

Fig. 9   einen Querschnitt III - III gem. Fig.6;

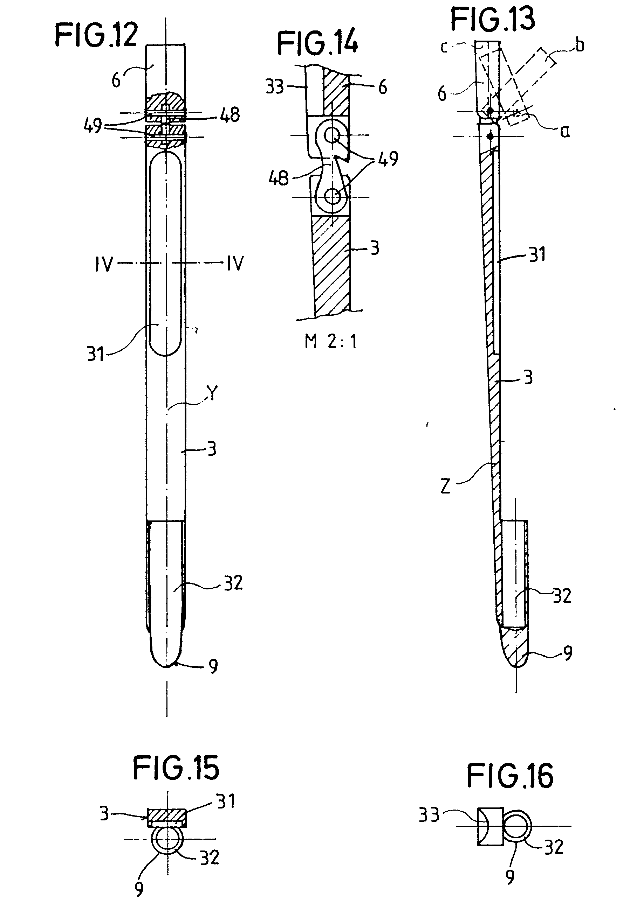

Fig. 10  einen Querschnitt III - III gem. Fig.6 in einer abgewandelten Ausführung;

Fig. 11  eine Ansicht des Schafthauptteils nach Fig.5
         von oben;

Fig. 12  eine Vorderansicht des keilförmigen Gegenstücks
         mit einer Teilschnittdarstellung;

Fig. 13  einen Längsschnitt durch das keilförmige Gegenstück nach Fig. 12;

Fig. 14  einen Ausschnitt aus Fig. 13 (Einzahndoppelgelenksperre) in vergrößerter Darstellung;

Fig. 15  einen Querschnitt IV - IV gem. Fig.12;

Fig. 16  eine Ansicht des keilförmigen Gegenstücks nach
         Fig. 13 von oben;

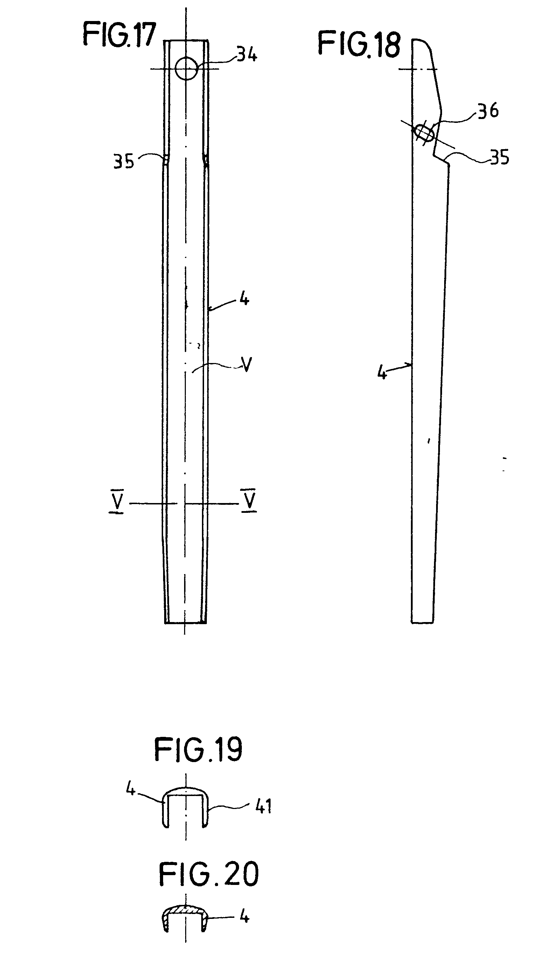

Fig. 17  eine Vorderansicht der Abdeckschniene;

Fig. 18  eine Seitenansicht der Abdeckschiene nach
         Fig.17;

Fig. 19    eine Ansicht der keilförmigen Abdeckschiene
           von oben;

Fig. 20    einen Querschnitt durch die keilförmige Abdeckschiene gem. V - V nach Fig.17;

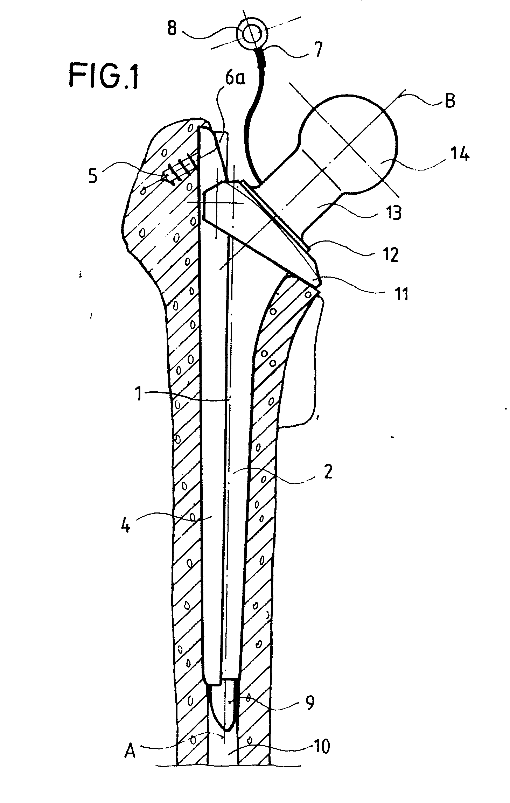

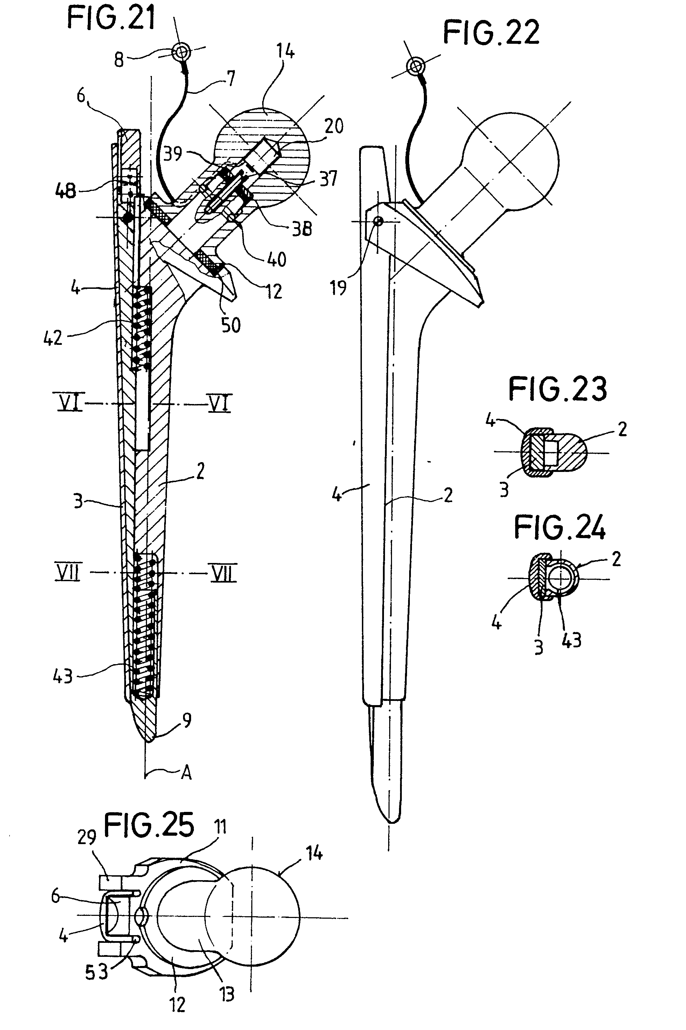

Fig. 21    einen Längsschnitt durch die Gelenkendoprothese nach Fig.1;

Fig. 22    eine Ansicht der Gelenkendoprothese nach -
           Fig.1 in ihrer Endposition;

Fig. 23    einen Querschnitt VI - VI nach Fig.21;

Fig. 24    einen Querschnitt VII - VII nach Fig.21;

Fig. 25    eine Ansicht der Gelenkendoprothese nach
           Fig. 21/22 von oben;

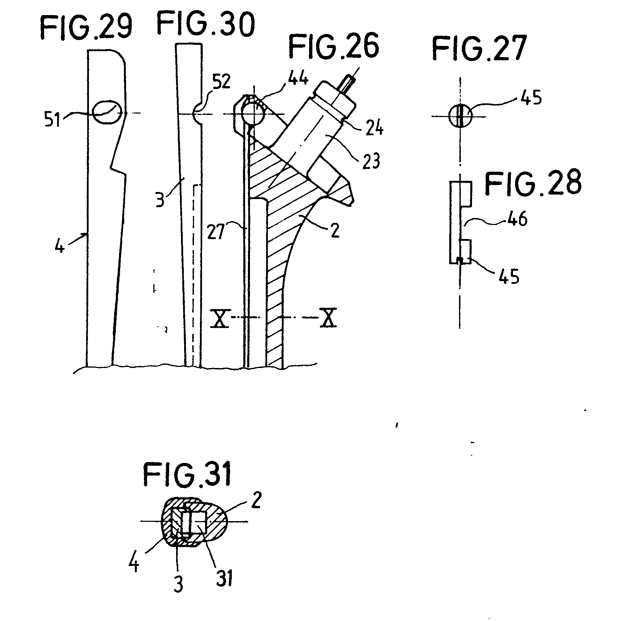

Fig. 26    einen Teil-Längsschnitt durch den Schafthauptteil in einer abgewandelten Ausführung;

Fig. 27    eine Stirnansicht und

Fig. 28    eine Seitenansicht des Zylinderstifts;

Fig. 29    eine Seitenansicht der Abdeckschiene in einer
           anderen Ausführung;

Fig. 30    eine Seitenansicht des keilförmigen Gegenstücks in einer anderen Ausführung;

Fig. 31    einen Querschnitt X - X durch den Schafthauptteil nach Fig.26 in zusammengebautem
           Zustand mit dem keilförmigen Gegenstück
           (Fig.30) und der Abdeckschiene (Fig.29);

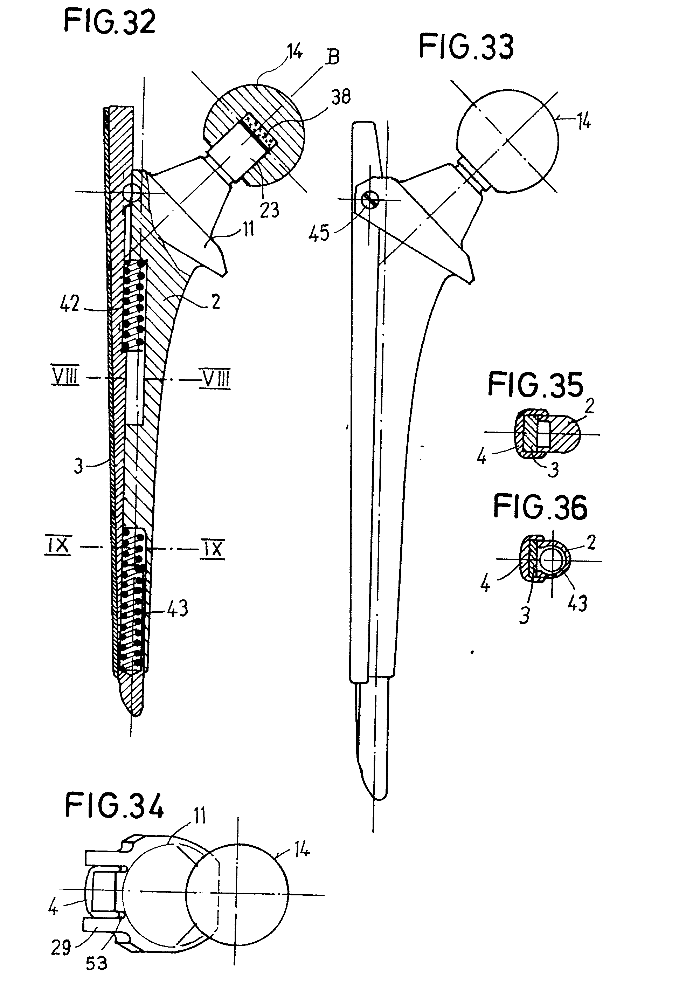

Fig. 32    einen Teil-Längsschnitt durch die Gelenkendoprothese in einer abgewandelten Ausführung;

Fig. 33    eine Seitenansicht der Gelenkendoprothese
           nach Fig. 32 in ihrer Endposition;

Fig. 34    eine Draufsicht nach den Fig. 32/33 auf eine
           zusammengebaute Endprothese;

Fig. 35    einen Querschnitt VIII - VIII nach Fig. 32
           sowie

Fig. 36    einen Querschnitt IX - IX nach Fig 32, jeweils durch einen gesamten Hüftgelenkschaft.

I-147 /EPA

Die Gelenkendoprothese als Hüftgelenkprothese ist in Fig. 1 in Seitenansicht, in den aufgeschnittenen Femurknochen eingesetzt, dargestellt, in welcher sie in "Betriebsstellung" ist, also in der entsicherten Ausgangsstellung nach der Implantierung; dabei steht das mit seinem nach unten herausragenden Vorstoßerteil sichtbare keilförmige Gegenstück unter Federdruck.

Der eigentliche Prothesenschaft 1 besteht aus Schafthauptteil 2, dem keilförmigen Gegenstück 3, das durch die Abdeckschiene 4 umhüllt ist. Die Nase des Gegenstückvorstoßers 9 bildet das Ende des eigentlichen Prothesenschaftes 1, der durch den Knochenmarkkanal 10 geführt wird. Der Schafthauptteil 2 endet in dem Prothesenkragen 11, der den Prothesenkopfsockel 12 umringt. Aus dem Prothesenhals 13 wird das Kabel 7 ausgeführt, das in einer ringförmigen Anschlußscheibe 8 endet. Mittellinie B des kugelförmigen Prothesenkopfes 14 trifft im vorliegenden Fall unter 45$^{\circ}$ Winkel auf die Mittellinie A des eigentlichen Prothesenschaftes 2. Durch die Spungiosaschraube 5 wird die Abdeckschiene 4 in seiner Position befestigt, wodurch auch das Übertragen von Rotations- sowie Druckkräften von Trochanter auf das Implantat und umgekehrt optimiert wird.

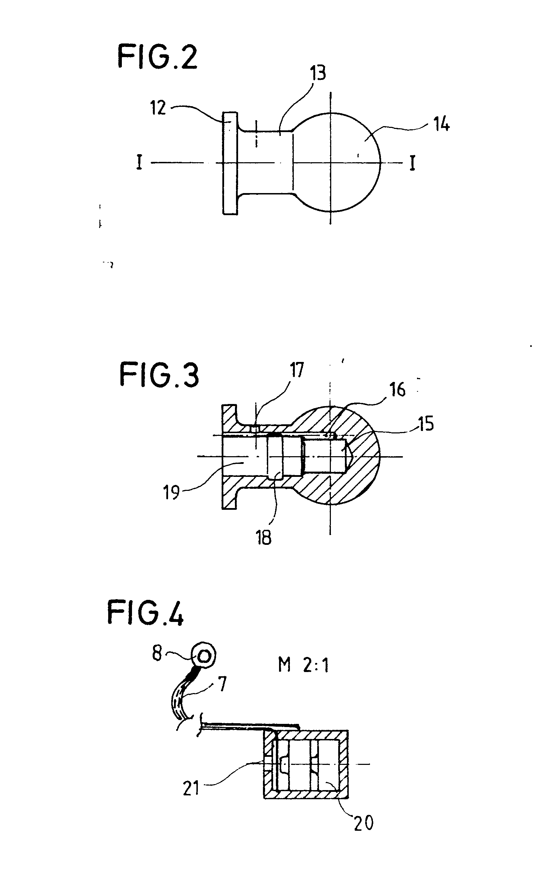

Die Fig. 2 zeigt den kugelförmigen Prothesenkopf 14, einen zylindrischen Hals 13, der im kreiszylindrischen Sockel 12 endet. Dabei entspricht die Achse I-I der Mittellinie B gemäß Fig. 1.

I-147 /EPA

Der Querschnitt gemäß Fig. 3 läßt die Bohrung 15 als Aufnahme für die Batterieeinheit 2o erkennen. Die Nut 16 stellt die Führung für das Kabel 7 dar und ist mit der Ausgangsbohrung 17 versehen. Ein breiter Einstich 18 dient als axiale Bewegungsbegrenzung gegenüber einer Ringfeder 40. Die Bohrung 19 ist die Aufnahme für den zylindrischen Einsteckbolzen 23.

In Fig. 4 ist die Batterieeinheit 20 im größeren Maßstab dargestellt, welche zwei Knopfzellen enthält. Die Kabel 7 führen zu der ringförmigen Anschlußscheibe 8. Eine Kontaktöffnung 21 dient zum Durchtritt der Feineinstellschraube 37.

Die Fig. 5 zeigt im Längsschnitt den Hauptteil 2 mit dem Kragen 11 der ringförmigen Senkung 22, aus deren Mitte der zylindrische Einsteckbolzen 23 herausragt. Die schmale Einstechnut 24 dient als Aufnahme für die Ringfeder 40. Die Bohrung 25 im Schafthauptteil 2 und die Nut 26 im oberen Teil des Schafthauptteils 3 dienen zur Aufnahme und als Widerlager der Druckfedern 42, 43, deren Achse parallel zur Schaftmittellinie A verläuft.

Die Darstellung Fig. 6 ist eine weitere Ansicht von Fig. 5, in der die aufgerauhte Auflagefläche 28 des Prothesenkragens 11 und die seitlichen Führungsnocken 29 sowie die Führungsnuten 53 für die Abdeckschiene 4 erkennbar sind.

Die Fig. 7 und 8 stellen jeweils einen Querschnitt II - II nach Fig. 6 dar. Es ist die Schaft-Federnut 26 im Schafthauptteil 2 zu erkennen. In Fig. 8 ist ferner eine weitere Nut 27 zur Führung des keilförmigen Gegenstücks 3 vorhanden.

Die Fig. 9 und 10 zeigen ebenfalls jeweils einen Querschnitt III - III durch den Schafthauptteil 2, jedoch weiter am unteren distalen Ende im Bereich des Vorstoßers 9, der das Schaft-Federwiderlager 25 in sich enthält. Die Fig. 10 zeigt den insoweit übereinstimmenden Querschnitt, der jedoch zusätzlich mit der Nut 27 als Führung des keilförmigen Gegenstücks 3 versehen ist.

Die Fig. 11 stellt eine Draufsicht der Fig. 6 dar und läßt die Führungsnut 53 für die Abdeckschiene 4 sowie eine Bohrung mit Gewinde 30 für eine Kontaktfeineinstellschraube 37 erkennen.

Die Fig. 12 zeigt eine Vorderansicht des keilförmigen Gegenstücks 3, dessen Fläche Y gepaart wird mit der Fläche X (siehe Zeichnung 3, Fig. 6) des Prothesenschafthauptteiles 2. Ferner ist der Sperrhebel 6 der Einzahndoppelgelenksperre und die Nut 31 als Federaufnahme im oberen Teil des keilförmigen Gegenstücks 3 zu erkennen, dessen Grundfläche parallel zu Mittellinie des eigentlichen Prothesenschaftes 1 verläuft. Der Vorstoßer des keilförmigen Gegenstücks 3 ist mit 9 bezeichnet.

Die Fig. 13 stellt einen Längsschnitt durch das Gegenstück 3 der Fig. 12 dar und zeigt den Sperrhebel 6 in drei Stellungen:

a) gesperrt

b) gesperrt, jedoch Einsetzung der Spongiosaschraube 5 möglich (siehe Zeichnung -1-)

c) entsicherte Stellung, in der der Sperrhebel 6 die Steigung des keilförmigen Gegenstücks 3 fortführt. Ferner ist das Federlager 32 im Vorstoßer 9 zur Druckfederaufnahme zu erkennen.

I-147/EPA

Die Einzahndoppelgelenksperre ist in Fig. 14 in einem größeren Maßstab dargestellt. Der Sperrhebel 6 ist mittels der Gelenksperrlasche 48 und den beiden Gelenkbolzen 49 am oberen Ende des Gegenstücks 3 derart angelenkt, daß er die drei in Fig. 13 dargestellten Stellungen a, b und c einnehmen kann. In der Stellung c ist das Gegenstück 3 entriegelt und kann unter der Wirkung des Federdrucks gleiten. In der Stellung b kann die Spongiosaschraube 5 eingeschraubt werden und die Stellung a ist die Verriegelungsstellung.

In der Schnittdarstellung Fig. 15 (Schnittverlauf IV - IV nach Fig. 12) ist die Federaufnahme 31 des keilförmigen Gegenstücks 3 zu erkennen, und ferner das Federlager 32 im Gegenstückvorstoßer 9.

Die Draufsicht nach Fig. 16 zeigt eine Halbrundnut 33 im Sperrhebel 6 sowie ebenfalls das Federlager 32 im Vorstoßer 9. Die Halbrundnut 33 dient zur Aufnahme des Kopfes der Sponiosaschraube 5, damit die Stellung c (Fig. 14) des Sperrhebels 6 erreicht werden kann.

Die Fig. 17 stellt die Abdeckschiene 4 dar, deren Fläche V mit der Fläche Z des keilförmigen Gegenstücks 3 gepaart wird (siehe Fig. 13). Im oberen Teil der Abdeckschiene 4 ist eine Bohrung 34 für die Spongiosaschraube 5 angeordnet. Die Anlagefläche 35 greift unter die Grundfläche 28 des Prothesenkragens 11.

In Fig. 18 ist eine weitere Ansicht der Abdeckschiene 4 dargestellt, die ein Langloch 36 aufweist, dessen Längsmittellinie im gleichen Winkel zur Längsmittellinie des eigentlichen Schaftes 1, wie die Grundfläche des Prothesenkragens 11 verläuft.

Die Fig. 19 zeigt eine weitere Ansicht der Abdeckschiene 4 gemäß Fig. 17 U-förmiges Profil und den seitlichen Flanken 41.

Die Fig. 20 ist eine Schnittdarstellung der Fig. 17 und zeigt das U-förmige Profil der Abdeckschiene 4, deren Außenflächen leicht schräg zur Mittellinie verlaufen. Alle Innenflächen verlaufen parallel zueinander.

Die Fig. 21 zeigt in einem Längsschnitt durch die Hüftgelenkprothese ihre erfindungsgemäßen Bauelemente in zusammengebautem Zustand in der Ausgangsstellung. In dem kugelförmigen Prothesenkopf 14 ist die Batterieeinheit 20 (siehe Fig. 4) untergebracht. Die Kontaktfeineinstellschraube 37 ist durch ein Gummiplättchen 38 umringt, das auf der Planfläche des zylindrischen Einsteckbolzens liegt und an der eine Unterlagsscheibe 39 ist. Das Gummiplättchen 38 hat die Aufgabe, die Kontaktfeineinstellschraube gegen Verstellen zu sichern, sowie eine druck- und stoßdämpfende Eigenschaft. Die Ringfeder 40, die in dem schmalen Einstich 24 des zylindrischen Einsteckbolzens 24 (siehe Fig. 5) eingesetzt ist, und beim Aufsetzen des Prothesenkopfes 14 in dem breiten Einstich 18 (siehe Fig. 3) einrastet, in dem Augenblick, wenn die Grundfläche des Sockels 12 den unter ihr liegenden Gummiring 50 berührt. Dadurch ist der zylindrische Einsteckbolzen 23 gegen Herausziehen aus dem Prothesenkopf 14 und umgekehrt gesichert.

I-147/EPA

Die genannten Elemente 12, 14, 20, 37, 38, 39 und 50 bilden zusammen einen Schalter. Wenn eine Kraft auf den Prothesenkopf 14 wirkt, wird sie zunächst durch die Elemente 38 und 50 gemindert und gedämpft, bevor sie über den eigentlichen Prothesenschaft 2 auf den ihn umschließenden Femurknochen übertragen wird. Übersteigt die auf den Prothesenkopf 14 wirkende Kraft die vorgesehene Belastungsobergrenze, so wird der Gummiring 50 sowie auch das Gummiplättchen 38 komprimiert, wodurch die Batterieeinheit 20 auf der Kontaktfeineinstellschraube 37 aufsitzt. Dadurch wird der Stromkreis geschlossen. Ist die Prothese implantiert, so wird der Strom über das Kabel 7 an die ringförmige Anschlußscheibe 8 geleitet, wodurch der Prothesenträger in der Umgebung der ringförmigen Anschlußscheibe einen Reiz verspürt. So kann er die genaue Belastungsgrenze, ohne Schaden zu nehmen, erlernen. Vorgesehenes Befestigungsgestell der ringförmigen Anschlußscheibe 8 ist unterhalb des Kopfes der Spongiosaschraube 5 (siehe Fig. 1).

Die Druckfeder 42 ist zu einem Teil ihres Durchmessers in der Nut 26 des Schafthauptteiles 2 untergebracht (siehe Fig. 5) und mit dem anderen Teil ihres Durchmessers in der Federaufnahme 31 des keilförmigen Gegenstücks 3 (siehe Fig. 12).

In der Ausgangsposition des keilförmigen Gegenstücks 3 decken sich die zwei eben genannten Vertiefungen nicht ab, wobei die Federaufnahme 31 des keilförmigen Gegenstücks 3 nach oben zu der Federnut 26 des Schafthauptteiles 2 versetzt ist, wodurch die in ihnen liegende Druckfeder 42 zusammengedrückt ist und daher

I-147 /EPA

auf das keilförmige Gegenstück 3 wirkt. Die Druckfeder 43 befindet sich im Federlager 32 des Gegenstücks mit Vorstoßer 9 (siehe Fig. 12 und 13). Die eben genannte Druckfeder 43 ist zusammengedrückt, wenn sich das keilförmige Gegenstück 3 in der Ausgangsposition befindet und wirkt mit niedrigerer Druckkraft als die Druckfeder 42 auf das keilförmige Gegenstück 3.

Der Sperrhebel 6 der Einzahndoppelgelenksperre befindet sich in der entsicherten Stellung c (siehe Fig. 13). Die Druckfedern 42 und 43 wirken auf das keilförmige Gegenstück 3. Tritt eine Prothesenschaftlockerung des im Femurknochen verankerten eigentlichen Prothesenschaftes 1 (siehe Fig. 1) auf, so bewegt sich das keilförmige Gegenstück 3 in durch den Federdruck der ihm vorgegebenen Richtung, bis die Paarungsflächen der Bauelemente des eigentlichen Prothesenschaftes miteinander und gegen die sie umgebenden harten Knochensubstanz verkeilt werden, wodurch das Weitergleiten des keilförmigen Gegenstücks 3 zum Stillstand kommt und dadurch die Lockerung durch Umfang- und Längenvergrößerung des eigentlichen Prothesenschaftes aufgehoben wird.

Die Fig. 22 zeigt die Prothese gegenüber dem Zustand in Fig. 21 in ihrer Endposition. Der eigentliche Prothesenschaft 1 hat durch die mechanische Flächenverschiebung selbsttätig seinen Umfang und Länge vergrößert, was einer Volumenvergrößerung des eigentlichen Schaftes gleichkommt.

I-147 /EPA

Die Fig. 23 und 24 zeigen Querschnitte VI - VI und VII - VII des Prothesenschaftes nach Fig. 21, in denen mit 2 der Schafthauptteil, mit 3 das Gegenstück und mit 4 die Abdeckschiene bezeichnet sind; ferner ist die Druckfeder 43 zu erkennen. In der Draufsicht auf die Prothese nach den Fig. 21 und 22 sind die einzelnen Teile wie in den Fig. 1 bis 6 bezeichnet.

In den Fig. 26 bis 36 ist eine abgewandelte Ausführungsform gezeigt, bei welcher ein Zylinderstift 45, siehe Fig. 27, die Abdeckschiene 4 durch das Langloch 51 mit dem Schafthauptteil 2 durch die Bohrung 44 verbindet. Durch Verdrehen des Zylinderstifes Fig. 27, 28 um $180^{o}$ wird das keilförmige Gegenstück 3, siehe Fig. 30, durch die Nut 46 in seiner Ausgangsstellung festgelegt oder entsichert. Der Prothesenkopf 14 wird gleichzeitig gegen Rotation gesichert.

Fig. 26 zeigt einen Längsschnitt durch das Schafthauptteil 2 mit den seitlichen Längsführungen 27 für das keilförmige Gegenstück 3 sowie die Bohrung 44 für die Aufnahme des Zylinderstiftes 45.

Die Fig. 27 und 28 stellen den Zylinderstift 45 in Vorder- und in Draufsicht dar. Der Schlitz dient zum Einsetzen von entsprechendem Werkzeug zum Verdrehen. Die Funktionsnut 46 dient zum Entriegeln aus der Halbnut 52 (Fig. 30).

Die Fig. 29 zeigt die Abdeckschiene 4 mit ihrem Langloch 51, dessen Längsmittellinie im Winkel von $90^{o}$ auf die Längsmittellinie des eigentlichen Schaftes trifft. In der Abdeckschiene 4 ist keine Bohrung für die Spongiosaschraube vorhanden.

In Fig. 30 ist das keilförmige Gegenstück 3 ohne die Doppelgelenksperre dargestellt. Die Funktion der Doppelgelenksperre wird dabei durch die Halbrundnut 52 ersetzt.

Die Fig. 31 zeigt einen Schnitt X - X nach Fig. 26, aus dem im Querschnitt der zusammengebaute Prothesenschaft ersichtlich ist. Der Schafthauptteil ist mit 2, das keilförmige Gegenstück mit 3 und die Abdeckschiene mit 4 bezeichnet. Der Hohlraum für die Druckfederaufnahme trägt das Bezugszeichen 31.

Die Fig. 32 bis 36 zeigen eine etwas vereinfachte Ausführungsmöglichkeit der Hüftgelenkprothese, deren Eigenschaften im wesentlichen gleich bleiben, jedoch ohne Überlastungssicherung.

Die Fig. 32 zeigt einen Teilschnitt durch den Prothesenschaft 1 und den Prothesenkopf 14, der kugelförmig ist und eine zylindrische Bohrung 19 für den Einsteckbolzen 23 aufweist. Auf der Planfläche des zylindrischen Einsteckbolzens 23 sitzt als dämpfende Einheit das Gummiplättchen 38. Der Hüftgelenkkopf 14 sitzt auf seinen Einsteckbolzen 23, der sich konisch nach allen Seiten erweitert und in den Kragen 11 übergeht. Der Prothesenkragen 11 geht in den Schafthauptteil 2 über, der sich gleichmäßig verjüngt. Die restlichen Bauelemente sind gleich wie in Fig. 21 dargestellt und beschrieben.

Fig. 33 zeigt eine Seitenansicht Hüftgelenkendoprothese in der Endstellung der Funktion;

P A T E N T A N S P R Ü C H E
================================

1. Gelenkendoprothese, insbesondere Hüftgelenkprothese, deren Schaft sich vom distalen freien Ende aus konisch erweitert und aus einem Schafthauptteil und einem lateralen, keilförmigen Gegenstück besteht, welches am Schafthauptteil längsverschiebbar ist,

dadurch gekennzeichnet,

daß das Gegenstück (3) unter einem in Richtung zum freien Ende wirksamen Druck von am Schafthauptteil (2) abgestützten Federelementen (42,43) steht und gegenüber dem Schafthauptteil (2) in seiner Ausgangsstellung lösbar verriegelt ist.

2. Gelenkendoprothese nach Anspruch 1, dadurch gekennzeichnet, daß das keilförmige Gegenstück (3) am Schafthauptteil (2) geführt und von einer Abdeckschiene (4) lateral und beidseitig umschlossen ist, wobei gegebenenfalls die Abdeckschiene (4) einen U-förmigen Querschnitt aufweist und das Gegenstück (3) umschließt sowie den Schafthauptteil (2) beidseitig teilweise umgreift oder das keilförmige Gegenstück (3) in einer Führungsnut (27) des Schafthauptteils (2) an diesem geführt ist.

-A 2-

3. Gelenkendoprothese nach Anspruch 1, dadurch gekennzeichnet, daß der Schafthauptteil (2) mit einem Schaft-Federwiderlager (25) und einer Schaft-Federnut (26) zur Aufnahme von Druckfedern (42,43) versehen ist, wobei gegebenenfalls das Gegenstück (3) mit einer Federaufnahme (31) und einem Federlager (32) versehen ist.

4. Gelenkendoprothese nach Anspruch 1, dadurch gekennzeichnet, daß mittels einer Einzahndoppelgelenksperre, bestehend aus einem Sperrhebel (6), der durch eine Gelenksperrlasche (48) und zwei Gelenkbolzen (49) mit dem Gegenstück (3) auslenkbar verbunden ist, wahlweise drei Ver- oder Entriegelungsstellungen des Sperrhebels (6) gegenüber dem Schafthauptteil (2) einstellbar sind, in denen beide Teile gegeneinander verriegelt sind, in einer zweiten Stellung das Einsetzen einer Spongiosaschraube (5) möglich ist, und in einer dritten Stellung das Gegenstück (3) entriegelt ist, so daß es unter der Federwirkung längsverschiebbar ist.

5. Gelenkendoprothese nach Anspruch 1, dadurch gekennzeichnet, daß der Schafthauptteil (2) in einem Prothesenkragen (11) endet, der den Prothesenkopfsockel (12) umringt und der eine ringförmige Senkung (22) aufweist, aus deren Mitte der zylindrische Einsteckbolzen (23) herausragt, der mit einer Ringfedernut (24) zur Aufnahme der Ringfeder (40) versehen ist, die zur Sicherung des Prothesenkopfs (14) gegen Herausziehen dient, wobei gegebenenfalls der Prothesenkopf (14) mit einem in die ringförmige Senkung (22) des Prothesenkragens (11) des Schafthauptteils (2) passenden Prothesenkopfsockel (12), mit einer hinteren Batterieaufnahmebohrung (15) und mit einer vorderen Einsteckbolzenbohrung (19) versehen ist, in welcher eine eingeschnittene breite Federringnut (18) angebracht ist,
die eine Axialbewegung des Prothesenkopfes (14)
gegenüber der in die Ringfedernut (24) des Einsteckbolzens (23) eingesetzten Ringfeder (40) ermöglicht.

6. Gelenkendoprothese nach Anspruch 5, dadurch gekennzeichnet, daß zwischen dem Prothesenkopfsockel (12)
und der Grundfläche der ringförmigen Senkung (22) im
Prothesenkragen (11) ein elastischer Gummiring (50)
eingesetzt ist.

7. Gelenkendoprothese nach Anspruch 5 oder 6, dadurch
gekennzeichnet, daß auf der Stirnfläche des Einsteckbolzens (23) ein Gummiplättchen (38) angeordnet ist
und/oder daß zwischen der Stirnfläche des Einsteckbolzens (23) und dem Gummiplättchen (38) eine Unterlegscheibe aus einem elektrisch leitenden Werkstoff,
und daß in eine Gewindebohrung (30) im Zentrum des
Einsteckbolzens (23) eine Kontaktfeineinstellschraube
(37) eingesetzt ist, mit welcher die in die Batterieaufnahmebohrung (15) des Prothesenkopfes (14) eingesetzte Batterieeinheit (20) bei stärkerer Zusammenpressung des elastischen Gummiringes (50) und
des Gummiplättchens (38) in Berührung gelangt.

8. Gelenkendoprothese nach einem der Ansprüche 5 bis 7, dadurch gekennzeichnet, daß die Kontaktfeineinstellschraube (37) durch Reibung in der engen Mittelbohrung des auf dem Einsteckbolzen (23) aufsitzenden Gummiplättchens (38) gegen ungewolltes Verdrehen gesichert ist, oder daß mittels Kabeln (7) der beim Berühren der Batterieeinheit (20) mit der Kontaktfeineinstellschraube (37) ausgelöste Stromstoß zu einem Signalgeber weiterleitbar ist.

9. Gelenkendoprothese nach Anspruch 7 oder 8, dadurch gekennzeichnet, daß als Signalgeber eine auf der Haut des Prothesenträgers befestigte oder aber implantierte Anschlußscheibe (8) dient, und/oder daß der Prothesenkopf (14) mit einer in die Batterieaufnahmebohrung (15) und die Einsteckbolzenbohrung (19) eingestochene Kabelnut (16) und einem in diese mündenden, nach außen führenden Kabelausgang (17) versehen ist.

10. Gelenkendoprothese nach einem der Ansprüche 1 bis 3, dadurch gekennzeichnet, daß im Bereich der oberen Enden des keilförmigen Gegenstücks (3) eine Halbrundnut (52) und der Abdeckschiene (4) ein in Querrichtung verlaufendes, mit der Halbrundnut (52) axial übereinstimmendes Langloch (51) zum Durchstecken des Zylinderstiftes (45) angebracht sind, der in einer Aufnahmebohrung (44) des Schafthauptteils (2) gelagert ist.

11. Gelenkendoprothese nach wenigstens einem der voraufgehenden Ansprüche, dadurch gekennzeichnet, daß das Abdeckelement den Schafthauptteil (2) an seinem Ende umschließt und beide um die Weglänge des keilförmigen Gleitelementes verlängert sind, so daß der zylindrische Vorstoßer (9) und dessen Nase innerhalb des eigentlichen Prothesenschaftes bewegbar sind.

12. Gelenkendoprothese nach wenigstens einem der Ansprüche 4 bis 11, dadurch gekennzeichnet, daß die Einzahndoppelgelenksperre weitere Einrastzähne aufweist.

FIG.1

0170982

-1/8-

FIG.2

FIG.3

FIG.4

M 2:1

FIG.5

FIG.6

FIG.11

FIG.7

FIG.8

FIG.9

FIG.10

FIG.12

6

49 — 48

IV —·—·— IV

31

Y

3

32

9

FIG.14

33 — 6

49

48

3

M 2:1

FIG.13

c — b

6

a

31

3

Z

32

9

FIG.15

3 — 31

32

9

FIG.16

33

32

9

FIG.17

34

35

4

V

V̄ ————— V̄

FIG.18

36

35

4

FIG.19

4

41

FIG.20

4

FIG.21

FIG.22

FIG.23

FIG.24

FIG.25

FIG.29   FIG.30   FIG.26   FIG.27

FIG.28

FIG.31

FIG.32

14 B

38

23

11

42

2

VIII VIII

3

IX IX

43

FIG.33

14

45

FIG.35

2

4

3

FIG.36

2

4

43

3

FIG.34

11

14

4

29

53

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 085 147 (GEBRÜDER SULZER AG et al.) <br> * Anspruch 1; Figuren * | 1 | A 61 F 2/36 |
| Y | DE-A-2 724 234 (HUGGLER) <br> * Seite 4, letzter Absatz - Seite 5, Zeile 5; Figur 1 * | 1 | |
| A | FR-A-1 278 359 (MAISON DRAPIER) <br> * Figuren 1-3 * | 1 | |
| A | GB-A-2 078 523 (LORD CORP.) <br> * Figur 1 * | 1 | |
| A | FR-A-2 290 880 (ETABLISSEMENTS DEAUX) <br> * Figur 1 * | 5 | RECHERCHIERTE SACHGEBIETE (Int. Cl.4) |
| A | DE-A-2 823 215 (HAUNI-WERKE KÖRBER & CO. KG.) <br> * Anspruch 1 * | 5 | A 61 F 2/00 |
| A | DE-A-2 247 721 (HOFFMANN-DAIMLER) | | |
| A | EP-A-0 046 926 (W. LINK GMBH & CO.) | | |

Der vorliegende Recherchenbericht wurde für alle Patentansprüche erstellt.

| Recherchenort | Abschlußdatum der Recherche | Prüfer |
|---|---|---|
| BERLIN | 18-10-1985 | KANAL P K |